(19) Europäisches Patentamt
European Patent Office
Office européen des brevets

(11) Publication number: 0 100 053
B1

(12) # EUROPEAN PATENT SPECIFICATION

(45) Date of publication of patent specification: 21.10.87

(21) Application number: 83107033.9

(22) Date of filing: 18.07.83

(51) Int. Cl.$^4$: C 07 C 153/07, C 07 D 295/14, C 07 D 217/26, C 07 D 307/52, C 07 D 333/16, C 07 D 333/22 // A61K31/21

(54) **Compounds having angiotensin converting enzyme inhibitory activity and diuretic activity.**

(30) Priority: 21.07.82 US 400555

(43) Date of publication of application: 08.02.84 Bulletin 84/06

(45) Publication of the grant of the patent: 21.10.87 Bulletin 87/43

(84) Designated Contracting States: AT BE CH DE FR GB IT LI LU NL SE

(56) References cited:
CH-A- 621 763
DE-A-3 044 236
DE-A-3 122 541
US-A-4 105 776
US-A-4 256 761

**The file contains technical information submitted after the application was filed and not included in this specification**

(73) Proprietor: RORER PHARMACEUTICAL CORPORATION
500 Virginia Drive
Fort Washington Pennsylvania 19034 (US)

(72) Inventor: Neiss, Edward S.
200 Charter Oak Drive
New Caanan Connecticut (US)
Inventor: Suh, John T.
59 Stanwich Road
Greenwich Connecticut (US)
Inventor: Piwinski, John J.
271 King Street
Port Chester New York (US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 100 053 B1

**Description**

BACKGROUND OF THE INVENTION

This invention relates to new chemical compounds combining angiotensin converting enzyme inhibitory activity and diuretic activity.

Compounds are known which are capable of suppressing or inhibiting the conversion of angiotensin I to angiotensin II, a property making them useful in the treatment of hypertension. Illustrative of such compounds are those disclosed in U.S. Patent No. 4,105,776 and having the general formula

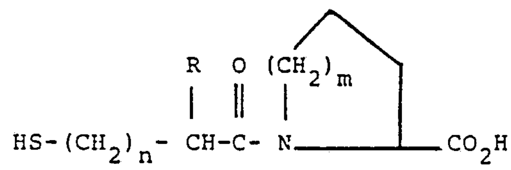

in which R is hydrogen or alkyl of 1 to 7 carbon atoms, preferably methyl, m is 1 or 2 and n is 0 or 1, and those disclosed in U.S. Patent No. 4,256,761 and having the general formula

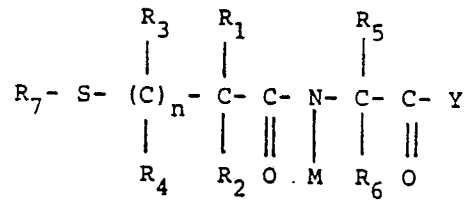

in which $R_1$, $R_2$, $R_3$, $R_4$, $R_5$, and $R_6$ are hydrogen, alkyl, alkenyl, alkynyl, phenyl-alkyl, and cycloalkyl, and may be the same or different; n is an integer from 0 to 4 inclusive; M is alkenyl, alkynyl, cycloalkyl, cycloalkyl-alkyl, polycycloalkyl, polycycloalkyl-alkyl, aryl, aralkyl, heteroaryl, heteroaryl-alkyl, hetero-cycloalkyl, hetero-cycloalky-alkyl, fused aryl-cycloalkyl, fused aryl-cycloalkyl-alkyl, fused heteroaryl-cycloalkyl, fused heteroaryl-cycloalkyl-alkyl, alkoxyalkyl, alkylthioalkyl, alkylamino-alkyl, or dialkylamminoalkyl; Y is hydroxy, alkoxy, amino, or substituted amino, aminoalkanoyl, aryloxy, aminoalkoxy, or hydroxyalkoxy; and, $R_7$ is hydrogen, alkanoyl, carboxyalkanoyl, hydroxy-alkanoyl, aminoalkanoyl, cyano, amidino, carbalkoxy, ZS, or

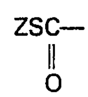

where Z is hydrogen, alkyl, hydroxyalkyl, or the radical

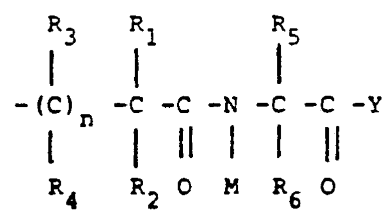

wherein $R_1$, $R_2$, $R_3$, $R_4$, $R_5$, $R_6$, n, M and Y are as described above; and where Y is hydroxy, pharmaceutically acceptable alkali metal, alkaline earth metal, and amine salts.

In addition, in commonly assigned U.S. patent 4311250 are described compounds having angiotension converting enzyme inhibitory activity of the following structure:

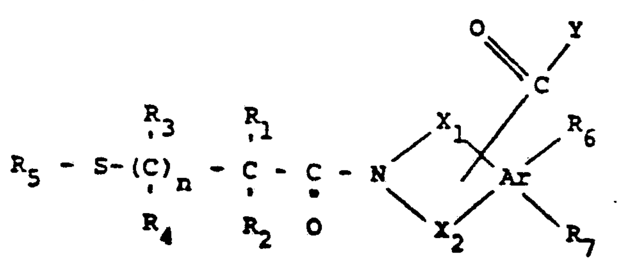

wherein $R_1$, $R_2$, $R_3$, $R_4$ are each hydrogen, alkyl, alkenyl, alkynyl, aryl, heteroaryl, aralkyl, heteroaralkyl, cycloalkyl, polycycloalkyl, or heterocycloalkyl;

n is an integer from 0 to 4 inclusive;

$R_5$ is hydrogen, alkyl, aralkyl, aryl, hydroxyalkyl, aminoalkyl, alkanoyl, aryloyl, arylalkanoyl, hydroxyalkanoyl, carboxyalkanoyl, aminoalkanoyl, cyano, amino, alkylamino, arylamino, amidino, alkylamidino, arylamidino, or ZS—, $ZS(CR_1R_2)_n$— or ZSCO— wherein Z is alkyl, aryl, aralkyl or a radical of the formula

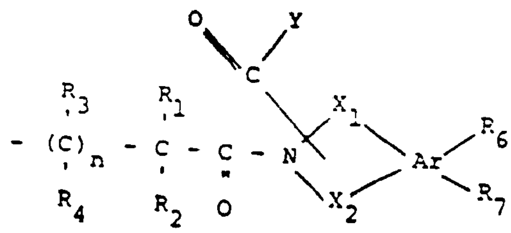

Y is OH, OM, $OR_1$, $NR_1R_2$, $—NR_1—(CR_1R_2)_n—CO—Y_1$

wherein M is a pharmaceutically acceptable cation;

$Y_1$ is OH, OM, $OR_1$ or $NR_1R_2$;

$X_1$ and $X_2$ are each S, SO, $—SO_2$, $NR_1$, chemical bond, O, $(CR_8{=}CR_9)_m$, $CR_8{=}CR_9$—, and CHOH, with the proviso that at least one of $X_1$ and $X_2$ can be $(CR_8R_9)_m$, wherein $R_8$ and $R_9$ are each hydrogen or lower alkyl, and m is an integer from 0 to 5;

Ar is a divalent arylene or heteroarylene; and

$R_6$ and $R_7$ are each hydrogen, alkyl, halo, cyano, hydroxy, alkoxy, amino, alkylamino, dialkylamino, mercapto, alkylmercapto, nitro, trifluoromethyl, carboxy, carbalkoxy, COY, and $NHCONHR_1$.

Numerous diuretics are known in the art including the benzene sulfonamides described in U.S. Patent No. 3,058,882 and having the general formula

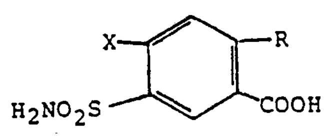

in which X represents a chlorine or bromine atom and R represents a benzylamino, dibenzylamino, furfurylamino or thenylamino group, and the salts thereof, and the (α-alkylidene acyl) phenoxy and (α-alkylidene acyl) phenyl-mercapto derivatives of monocarboxylic acids described in U.S. Patent No. 3,225,241 and having the general formula

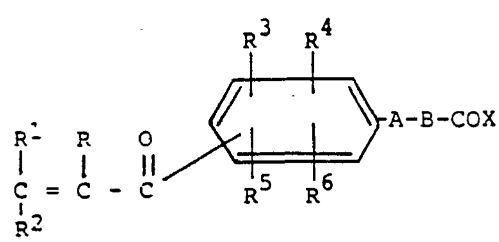

in which R, $R^1$ and $R^2$, respectively, is selected from the group consisting of hydrogen; halogen or halogen-like radicals; hydroxyl, lower aliphatic, lower aliphatic-oxy or lower aliphatic-thio, straight or branched chain, saturated or unsaturated, and unsubstituted or substituted, the substituent group(s) being alkyl, amino, halogen-like, carboxyl or substituted carboxyl, cyano, hydroxyl, alkylthio, arylthio, arylsulfonyl, alkylsulfonyl, nitro, and the like; alicyclic, either unsubstituted or substituted, the substituent groups being the same as those described above for the aliphatic group; aryl or aryloxy or aryl-thio, especially phenyl, phenoxy or phenylthio, wherein the aryl-(phenyl) moiety can be unsubstituted or a substituent can be attached to one or more of its carbon atoms selected advantageously from lower, straight or branched chain-alkyl, alkoxy, alkylthio, hydroxyl, halogen or halogen-like; arylaliphatic, especially a mononuclear-arylaliphatic, advantageously phenalkyl which can be attached through an oxygen or a sulfur atom to the grouping

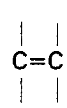

and which is either unsubstituted or substituted in the aryl and/or alkyl portions by substituents of the aforesaid type; cyano; and wherein R and $R^2$ additionally can be linked together to form preferably a 5 to 6

carbon ring with the carbons to which they are attached; $R^3$, $R^4$, $R^5$ and $R^6$ respectively can represent the same or different group selected from hydrogen, halogen, lower alipathic, straight or branched chain; lower aliphatic-oxy or lower aliphatic-thio, straight or branched chain, unsubstituted or having a substituent of the type described above for attachment to the lower aliphatic group; and wherein the lower aliphatic-oxy or lower aliphatic-thio is advantageously lower alkoxy or lower-alkylthio, unsubstituted or substituted, as for example, carboxyalkoxy, carboxyalkylthio, and the like; aliphaticsulfonyl, especially an alkylsulfonyl; hydroxy; nitro; amino-carboxy or substituted carboxy, especially carbamoyl and N-substituted carbamoyl; aryl, especially phenyl unsubstituted or substituted as described above for attachment to aryl (phenyl) moieties; or wherein $R^3$ and $R^4$ and/or $R^5$ and $R^6$ can additionally be linked together to form, with the ring carbons to which they are attached, a 5- or 6-membered carbocyclic ring; A represents oxygen or sulfur which can be oxidized to the sulfoxide or sulfone; B represents a divalent aliphatic, aromatic or aliphatic-aromatic group, preferably a straight or branched chain lower aliphatic group which can contain oxygen or sulfur atoms as part of the aliphatic chain; a phenyl-lower alkyl, or a phenyl group; X represents hydroxyl or salts of the resulting acids, i.e., metal salts, especially sodium, potassium, calcium and the like or amino salts; alkoxy, unsubstituted or substituted, the substituent(s) being dialiphatic amino and the like; amino such as an amino group of the structure $—NR^7R^6$ wherein $R^7$ and $R^6$ are the same or different; aliphatic, unsubstituted or substituted, group or aromatic, unsubstituted or substituted, group, especially substituted phenyl or $R^7$ and $R^6$ can be joined together to form, with the nitrogen atom to which they are attached, a hetero ring containing one or more hetero atoms as morpholinyl, piperazinyl, pyrrolidyl and the like; and, hydrazine, unsubstituted or substituted, advantageously with lower-alkyl group or groups.

DE—A1—3122541 and CH—A—5—621763 both disclose compounds similar to the present invention possessing angiotensin activity, the main difference being the inclusion of substituents on the backbone phenyl group in the present invention.

Unlike the compounds of the prior art, *supra,* which possess either angiotensin converting enzyme inhibitory activity or diuretic activity, the compounds of the present invention combine both activities in a single molecular species. The compounds of this invention are represented by the general formula

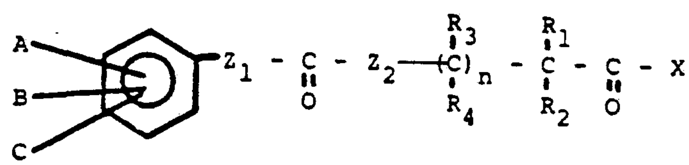

(I)

in which X is

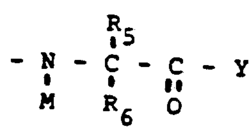

$R_1$, $R_2$, $R_3$, $R_4$, $R_5$, and $R_6$ are hydrogen, alkyl, alkenyl, alkynyl, phenyl-alkyl, and cycloalkyl, and may be the same or different;

n is an integer from 0 to 4 incluive;

M is an alkenyl, alkynyl, cycloalkyl, cycloalkyl-alkyl, polycycloalkyl, polycycloalkyl-alkyl, or M and $R_6$ taken together with the nitrogen to which M is bonded and the carbon to which $R_6$ is bonded form a saturated unsubstituted heterocyclic group containing 3 or 4 ring carbon atoms;

Y is hydroxy, alkoxy, amino, or substituted amino, $Z_1$ and $Z_2$ each is a chemical bond, the group $X_3—(CH_2)_m—$, an amino acid group or the group $X_3—(CH_2)_m—X_3$ wherein $X_3$ is oxygen, sulfur or NH and M is an integer from 0 to 4 inclusive;

A and B each is the same as $R_1$ or halogen, trifluoromethyl, $OR_1$, $NO_2$, $NR_1R_2$, $SO_2NH_2$, or $SR_1$;

C is hydrogen alkyl, amino, $—CR_1{=}CR_2$,

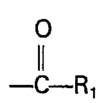

aminoalkyl, halogen, 2-furanylmethylamino, aminoaryl or aminobenzyl with the proviso that when $Z_2$ is sulfur and $Z_1$ is a chemical bond, then A, B and C cannot all be hydrogen; and salts, particularly pharmaceutically acceptable salts thereof.

DESCRIPTION OF THE PREFERRED EMBODIMENTS

Preferred compounds of the present invention are those of the above general formula in which $R_1$, $R_2$, $R_3$ and $R_4$ each is hydrogen or a lower alkyl group, preferably methyl; n is 1; M is cycloalkyl; one of $X_1$ and

$X_2$ is —$CH_2$— and the other is —$CH_2$—$CHR_1$, Ar is 0-phenylene; Y is hydroxy or alkoxy; $Z_1$ is a chemical bond, or the group —$OCH_2$—; $Z_2$ is sulfur; A and B each is hydrogen, halogen or the group —$SO_2NH_2$; and C is halogen, amino,

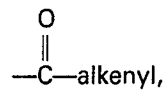

aminoalkyl, 2-furanylmethylamino, aminoaryl or aminobenzyl.

The alkyl groups per se and in the alkyl moiety in aralkyl, cycloalkylalkyl, polycycloalkylalkyl or heteroarylalkyl, and, in alkoxy, alkylmercapto, alkanoyl, carbalkoxy, alkylamino and dialkylamino, may be straight chained or branched and are preferably alkyl groups containing from 1 to 20 carbons. Such groups include methyl, ethyl, propyl, isopropyl, butyl, isobutyl, amyl, iso-amyl, hexyl, octyl or dodecyl. Preferably the alkyl groups are lower alkyl containing from 1 to 6 carbon atoms.

The alkenyl and alkynyl groups may also be branched or straight chained and contain from 2 to 6 carbon atoms. Such groups include vinyl, ethynyl, propenyl, allyl or isopropenyl.

The alkyl, alkenyl, and alkynyl groups may carry substituents such as hydroxy, alkoxy, amino, alkylamino, dialkylamino, mercapto, alkylmercapto or halo.

The cycloalkyl, polycycloalkyl, aryl, heteroaryl, arylalkyl, or fused aryl-cycloalkyl groups containing from 3 to 16 carbon atoms and may carry substituents such as lower alkyl, alkenyl, alkynyl, hydroxy, mercapto, amino, alkoxy, alkylmercapto, alkylamino, dialkylamino, halo or trifluoromethyl. The groups include such radicals as cyclopropyl, cyclobutyl, cyclopentyl, cyclohexyl, cycloheptyl, adamantyl, norbornyl, phenyl, tolyl, benzyl, penethyl, dimethoxyphenyl, hydroxybenzyl, indanyl, naphthyl, tetrahydronapthyl, decanhydronaphthyl, pyridyl, quinolyl, pyrrolidyl, pyrrolyl, morpholinyl, furyl, furfuryl, tetrahydrofurfuryl, benzimidazolyl, thienyl or imidazolyl.

The halo groups include fluoro, chloro, bromo and iodo.

Compounds in accordance with the present invention are readily prepared employing knowing starting materials and procedures. It will be understood by those skilled in the art that compounds having an asymmetric carbon may exist in both levo and dextro forms, said forms being within the scope of the invention.

Accordingly, the present invention comprises also a method for preparing a compound of formula (I) which comprises reacting under acylating conditions an acylating derivative of an acid of the formula

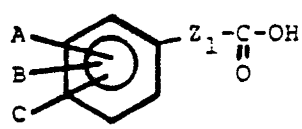

(II)

with a compound of the formula

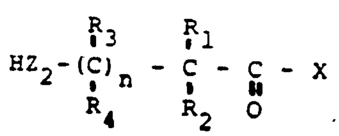

(III)

or, alternatively, the present new compounds can be prepared by reacting an acylating derivative of an acid of the general formula

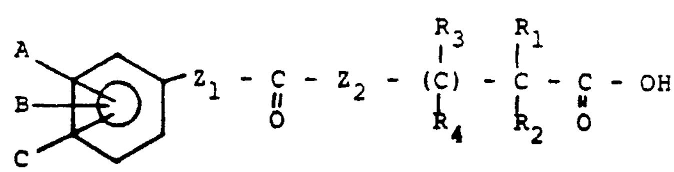

(IV)

with a compound of the formula

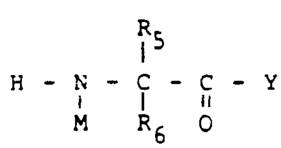

(V)

wherein X, $R_1$, $R_2$, $R_3$, $R_4$, $R_5$, $R_6$, n, M, $Z_1$, $Z_2$, A, B and C have the meanings as defined with respect to formula (I) and optionally forming salts of the resulting products, especially pharmaceutically accept salts thereof. Preferably, the reaction is carried out in the presence of carbodiimides as are commonly used in

peptide synthesis, e.g., N,N-dicyclohexylcarbodiimide. A reaction solvent is normally used to permit more initimate contact of the reactants and includes such solvents as ethers or ethylene glycol and diethylene glycol, tetrahydrofuran, dioxane, alkyl ketones such as acetone, methyl isobutyl ketone and similar solvents. Reaction temperature is not critical since the reaction proceeds smoothly at room temperature and even at lower temperatures. The use of high temperature for the reaction, e.g., reflux temperature of the reaction mixture, will shorten the reaction time but may lead to undesirable by-products. Generally, the reaction is allowed to proceed at room temperature until complete. The products are recovered by usual methods and purified as by recrystallization from suitable solvents.

In those compounds where free acid groups are present, e.g., where Y is OH, the compounds can readily form salts with suitable cations, preferably those which are pharmaceutically acceptable, e.g., both mono and polyvalent metal ions such as sodium, potassium, calcium, magnesium or iron, and ammonium ions derived from ammonia, primary secondary and tertiary amines.

The following examples are illustrative of the present invention.

## Example 1

This example illustrates the preparation of intermediates used in the preparation of compounds of this invention.

A. N-Cyclopentylglycine t-butyl ester

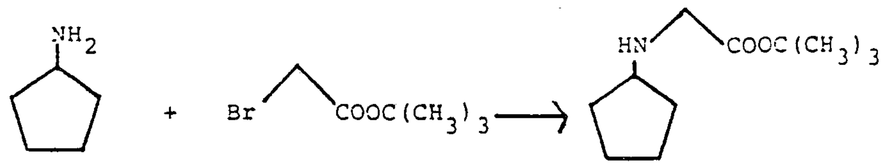

To a solution containing 75 g (0.88 mol) of cyclopentylamine and 100 g (1.0 mol) of triethylamine in 500 ml of ether at 0°C was slowly added 136 g (0.70 mol) of t-butyl bromoacetate in 200 ml of ether. After stirring at room temperature for 6 hours, the mixture was filtered, washed with water, and concentrated *in vacuo.* The residue was acidified with 5% aqueous HCl, washed with ether, rendered basic with 5% aqueous $K_2CO_3$, and extracted with ether. The ethereal solution was then washed with water, dried over $MgSO_4$, and concentrated *in vacuo* to give 90 g (65%) of N-cyclopentylglycene t-butyl ester as an oil which crystallized on standing.

B. N-(3-Acetylthio-2-methylpropanoyl)-N-cyclopentylglycine t-butyl ester

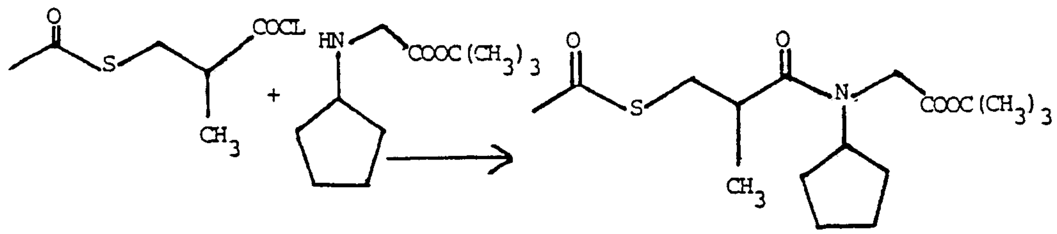

To a solution containing 60 g (0.30 mol) of N-cyclopentylglycine t-butyl ester and 75 ml (0.54 mol) of triethylamine in 200 ml of methylene chloride at 0°C was added dropwise a solution of 54 g (0.30 mol) of 3-acetylthio-2-methylpropanoyl chloride in 250 ml of methylene chloride. After the reaction mixture was stirred at room temperature overnight, it was filtered and concentrated *in vacuo.* The residue was taken up in ethyl acetate and washed once with saturated aqueous $NaHCO_3$, once with brine, three times with 5% aqueous HCl, once with brine, and five times with saturated aqueous $NaHCO_3$. The organic phase was dried over $MgSO_4$, filtered, and concentrated *in vacuo* to yield 80.1 g (78%) of N-(3-acetylthio-2-methylpropanoyl)-N-cyclopentylglycine t-butyl ester as an oil which crystallized on standing.

C. N-(3-Acetylthio-2-methylpropanoyl)-N-cyclopentyl glycine

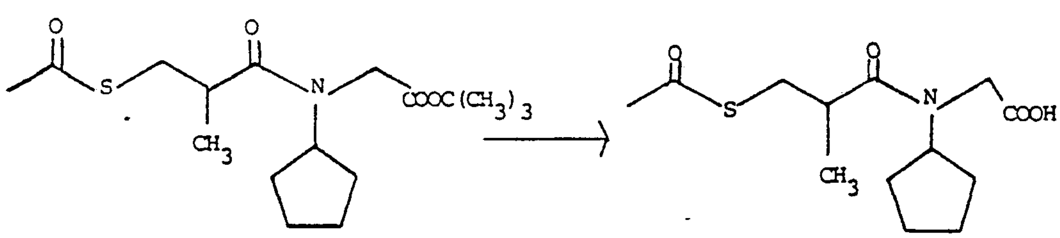

A mixture containing 80.1 g (0.23 mol) of N-(3-acetylthio-2-methyl-propanoyl)-N-cyclopentyl glycine t-butyl ester, 37.9 g (0.35 mol) of trimethylsilyl chloride, and 52.5 g (0.35 mol) of sodium iodide in 300 ml of

acetonitrile was heated between 45—50°C for 30 minutes. Following the adition of 50 ml of water, the mixture was concentrated *in vacuo.* The residue was rendered basic with 400 ml of saturated aqueous $NaHCO_3$, washed three times with ethyl acetate, acidified with concentrated HCl, and extracted three times with ethyl acetate. These later organic extracts were combined, dried over $MgSO_4$, filtered, and concentrated *in vacuo* to afford 60 g (91%) of the crude product. Purification via high pressure liquid chromatography (HPLC) [ethyl acetate/hexane/acetic acid (60/40/2)] afforded N-(3-acetylthio-2-methylpropanoyl)-N-cyclopentyl glycine as an oil.

D. N-(3-Mercapto-2-methylpropanoyl)-N-cyclopentyl glycine

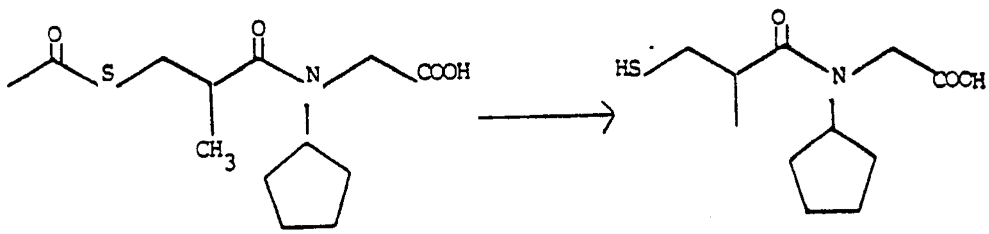

Into a solution containing 10.4 g (36 mmol) of N-(3-acetylthio-2-methylpropanoyl)-N-cyclopentyl glycine was bubbled ammonia gas for 1 hour. After 20 minutes the mixture was concentrated *in vacuo,* and the residue taken up in ethyl acetate and washed three times with 5% aqueous sodium bisulfate. It was then dried over $MgSO_4$, filtered, concentrated *in vacuo,* and purified via HPLC [ethyl acetate toluene/hexane/acetic acid (50/25/50/21)] to give 7.9 g (90%) of N-(3-mercapto-2-methylpropanoyl)-N-cyclopentyl glycine as an oil.

E. N-(3-Mercapto-2-methylpropanoyl)-N-cyclopentyl glycine methyl ester

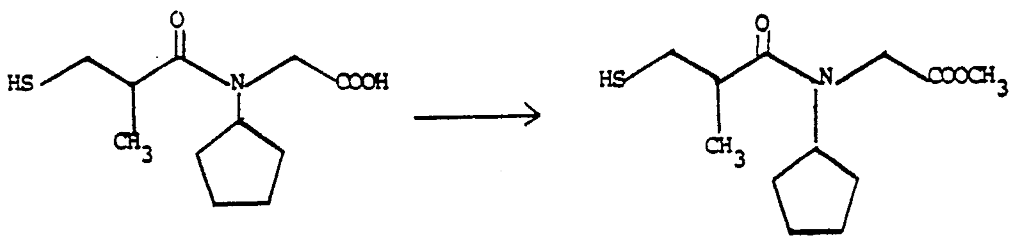

A mixture containing 19.0 g (77 mmol) of N-(3-mercapto-2-methylpropanoyl)-N-cyclopentyl glycine and 2.0 g (10.5 mmol) of p-toluene sulfonic acid in 500 ml of methanol was refluxed overnight. The reaction mixture was concentrated *in vacuo* and the residue taken up in ether. It was washed once with saturated aqueous sodium bicarbonate and once with brine. The organic phase was dried over $MgSO_4$, filtered, and concentrated *in vacuo* to afford N-(3-mercapto-2-methylpropanoyl)-N-cyclopentyl glycine methyl ester as an oil.

F. N-(3-Mercapto-2-methylpropanoyl)-N-cyclopentylglycine t-butyl ester

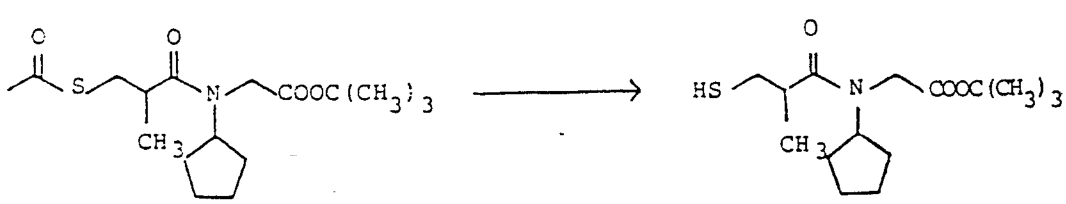

Into a solution containing 50 g (0.14 mol) of N-(3-acetylthio-2-methylpropanoyl)-N-cyclopentylglycine t-butyl ester in 700 ml of methanol was bubbled nitrogen gas for 10 minutes followed by ammonia gas for 30 minutes. After 30 minutes the mixture was concentrated *in vacuo* and the residue taken up in ethyl acetate. It was washed once with 3% aqueous HCl and once with water, dried over $MgSO_4$, filtered, and concentrated *in vacuo* to yield an oily product. Purification via HPLC [15% ethyl acetate in hexanes] afforded 30 g (70%) of N-(3-mercapto-2-methylpropanoyl)-N-cyclopentylglycine t-butyl ester as an oil.

Example 2

N-[3-[2,3-Dichloro-4-(2-methylene-1-oxobutyl) phenoxy] acetylthio-2-methylpropanoyl]-N-cyclopentyl glycine methyl ester

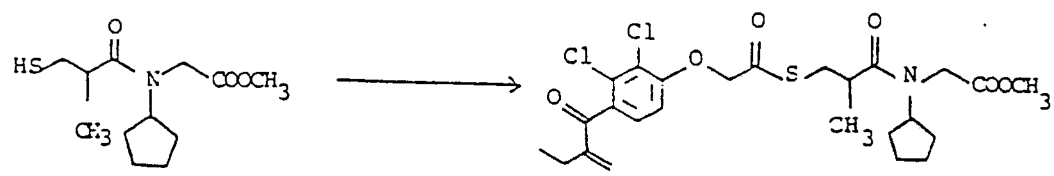

To a solution containing 10.5 g (40.0 mmol) of N-(3-mercapto-2-methylpropanoyl)-N-cyclopentyl glycine methyl ester and 12.1 g (39.9 mmol) of ethacrynic acid in 200 ml of glyme at 0°C was added a solution of 8.24 g (39.9 mmol) of N,N-dicyclohexylcarbodiimide in 20 ml of glyme. The solution was allowed to warm to room temperature and to stir overnight. The mixture was filtered and the filtrate concentrated *in vacuo*. Purification of the product by HPLC [hexane/ethyl acetate/acetone (70/20/15)] afforded N-[3-[2,3-dichloro-4-(2-methylene-1-oxobutyl) phenoxyl] acetylthio-2-methylpropanoyl]-N-cyclopentyl glycine methyl ester as a viscous oil.

Example 3

N-[3-[2,3-Dichloro-4-(2-methylene-1-oxobutyl)phenoxy] acetylthio-2-methylpropanoyl]-N-cyclopentyl glycine t-butyl ester

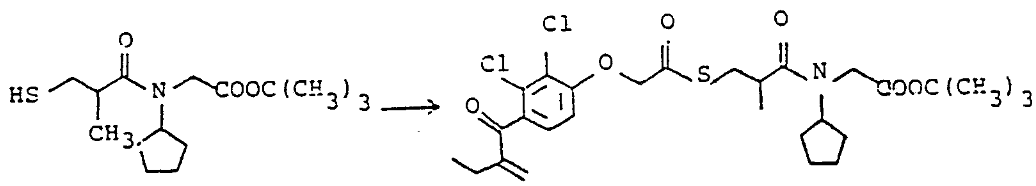

To a solution containing 19.2 g (63.8 mmol) of N-(3-mercapto-2-methylpropanoyl)-N-cyclopentyl-glycine t-butyl ester and 19.3 g (63.7 mmol) of ethacrynic acid in 200 mol of methylene chloride at 0°C was added a solution of 13.2 g (64.0 mmol) of N,N-dicyclohexylcarbodiimide in 20 ml of methylene chloride. The solution was allowed to warm to room temperature and to stir overnight. The solution was filtered and the filtrate was concentrated *in vacuo*. Purification of the product by HPLC [10% ethyl acetate in hexanes] afforded N-[3-[2,3-dichloro-4-(2-methylene-1-oxobutyl)phenoxy]acetylthio-2-methylpropanoyl]-N-cyclopentyl glycine t-butyl ester as a viscous oil.

Example 4

N-[3-[2,3-Dichloro-4-(2-methylene-1-oxobutyl)phenoxyl]acetylthio-2-methylpropanoyl]-N-cyclopentyl glycine

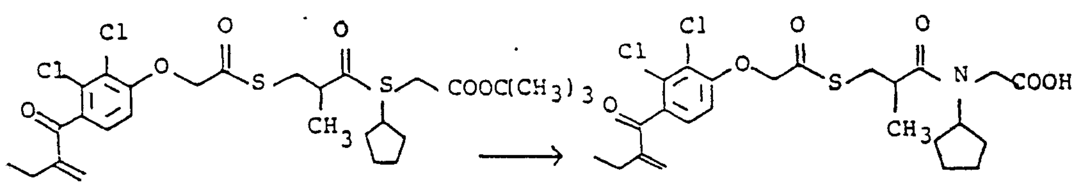

To a solution containing 5.2 g (8.7 mmol) of N-[3-[2,3-dichloro-4-(2-methylene-1-oxobutyl)phenoxyl] acetylthio-2-methylpropanoyl]-N-cyclopentyl glycine t-butyl ester in 100 ml of methylene chloride was added 9.0 ml (117 mmol) of trifluoroacetic acid. After 12 hours the reaction mixture was carefully concentrated *in vacuo* and the residue was chromatographed via HPLC [methylene chloride/ether/acetic acid (88/10/2.5)] to afford 2.4 g (52%) of N-[3-[2,3-dichloro-4-(2-methylene-1-oxobutyl)phenoxyl]acetylthio-2-methylpropanoyl]-N-cyclopentyl glycine as a glass.

Example 5

N-[3-(4-chloro-2-[(2-furanylmethyl)amino]-5-sulfamoyl)benzoylthio-2-methylpropanoyl]-N-cyclopentyl glycine t-butyl ester

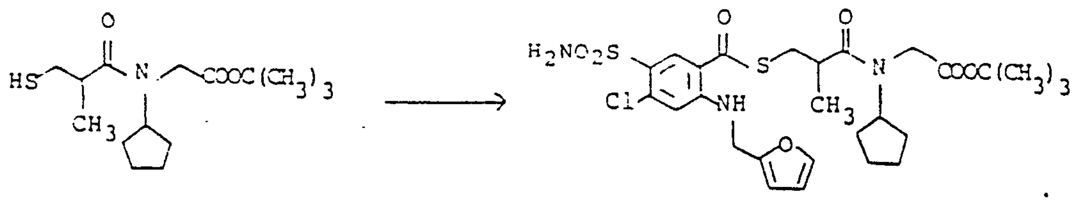

To a solution containing 11.5 g (38.2 mmol) of N-(3-mercapto-2-methyl propanoyl)-N-cyclopentylglycine t-butyl ester and 12.6 g (38.1 mmol) for furosemide in 100 ml of tetrahydrofuran and 35 ml of acetone at 0°C was added 7.84 g (38.0 mmol) of N,N-dicycloehexylcarbodiimide in 20 ml of tetrahydrofuran. The solution was slowly allowed to warm to room temperature and to stir overnight. The solution was filtered and the filtrate was concentrated *in vacuo*. Purification of the product by HPLC [Hexane/ethyl acetate/acetone (4/1/1)] afforded N-[3-(4-chloro-2-[(2-furanylmethyl) amino]-5-sulfamoyl)benzoylthio-2-methylpropanoyl]-N-cyclopentyl glycine t-butyl ester as a glass.

Example 6

N-[3-(4-chloro-2-[(2-furanylmethyl)amino]-5-sulfamoyl)benzoylthio-2-methylpropanoyl]-N-cyclopentyl glycine

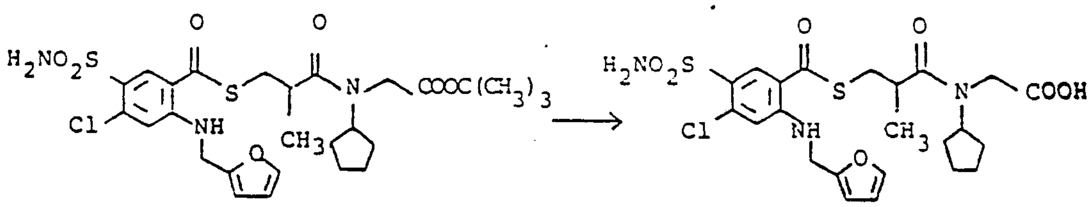

Into a solution containing 7.72 g (7.69 mmol) of N-[3-(4-chloro-2-[(2-furanylmethyl)amino]-5-sulfamoyl)benzoylthio-2-methylpropanoyl]-N-cyclopentyl glycine t-butyl ester and 624 ml (7.72 mmol) of pyridine in 100 ml of methylene chloride at −10°C was bubbled isobutylene for 5 minutes. To this solution was then added dropwise 2.03 ml (14.3 mmol) of trimethylsilyl iodide. After 2 hours the reaction was quenched by the addition of 1.0 ml of water. The solution was concentrated *in vacuo* and the residue was taken up in a chloroform/methylene chloride mixture. The precipitated solid was filtered off and recrystallized two times from an acetonitrile/ethanol/water mixture to afford 1.2 g (28%) of N-[3-(4-chloro-2-[(2-furanylmethyl)amino]-5-sulfamoyl)benzoylthio-2-methylpropanoyl]-N-cyclopentyl glycine as a yellow solid: M.P. 204—206°C.

In the same manner the mercaptan compounds in Column A are acylated by the acids in Column B to provide the compounds in Column C after cleavage of their t-butyl esters.

| A<br>Mercaptan | B<br>Actylated Acid | C<br>Product |
|---|---|---|
| 1. N-(3-mercapto-2-methylpropanoyl)-N-cyclopentyl glycine t-butyl ester | bumetanide | N-[3-(5-butylamino-4-phenoxy-3-sulfamoyl) benzoylthio-2-methyl-propanoyl]-N-cyclopentyl glycine |
| 2. N-(3-mercapto-2-methylpropanoyl)-N-cyclopentyl glycine t-butyl ester | piretanide | N-[3-(4-phenoxy-5-pyrrolidinyl-3-sulfamoyl)benzoylthio-2-methylpropanoyl)-N-cyclopentyl glycine. |
| 3. N-(3-mercapto-2-methylpropanoyl)-N-cyclopentyl glycine t-butyl ester | xipamide | N-(3-(4-chloro-2-hydroxy-5-sulfanoyl)benzothio-2-methylpropanoyl)-N-cyclopentyl glycine. |
| 4. N-(3-mercapto-2-methylpropanoyl)-N-cyclopentyl glycine t-butyl ester | 4-benzoyl-3-sulfamoyl-5-(3-thienyl-methyloxy)benzoic acid | N-[3-(4-benzoyl-3-sulfamoyl-5-(3-thienyl-methyloxy)]benzoylthio-2-methylpropanoyl]-N-cyclopentyl glycine. |
| 5. N-(3-mercapto-2-methylpropanoyl)-1,2,3,4-tetrahydroisoquinoline-3-carboxylic acid t-butyl ester | tienilic acid | N-[3-(2-dichloro-4-(2-thienylcarbonyl) phenoxy]acetylthio-2-methylpropanoyl]-N-cyclopentyl glycine. |
| 6. N-(3-mercapto-2-methylpropanoyl)-1,2,3,4-tetrahydroisoquinoline-3-carboxylic acid t-butyl ester | 2-(2-furfurylamino)-4-phenoxy-5-sulfamoyl-benzoic acid | N-3-[2-(2-furfurylamino)-4-phenoxy-5-sulfamoyl]-benzoylthio-2-methyl-propanoyl]-N-cyclopentyl glycine. |

5-Sulfamyl-4-chloro-2-[(2′-furanylmethyl)amino]benzoic acid is reacted with mercaptan compound #1 to form the corresponding acylated mercaptan. Similarly, 5-sulfamyl-2-[(2′-furanylmethyl)amino]-4-phenoxybenzoic acid with mercaptan compound #2 and 2,3-dichloro-4-(2-methylene-1-oxobutyl)-phenoxyacetic acid with compound #5 forms acylated mercaptans.

**Claims for the Contracting States: BE CH DE FR GB IT LI LU NL SE**

1. Compounds of the general formula

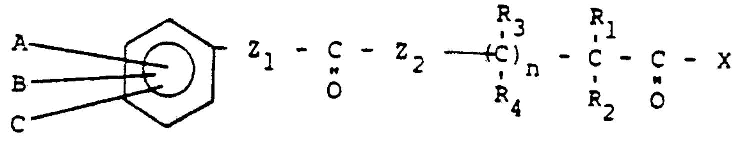

(I)

wherein X is

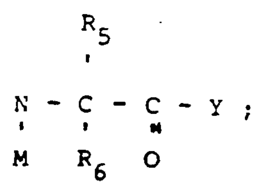

;

$R_1$, $R_2$, $R_3$, $R_4$, $R_5$, and $R_6$ are hydrogen, alkyl, alkenyl, alkynl, phenyl-alkyl, and cycloalkyl, and may be the same or different;

n is an integer from 0 to 4 inclusive;

M is alkenyl, alkynyl, cycloalkyl, cycloalkyl-alkyl, polycycloalkyl, polycycloalkyl-alkyl, or M and $R_6$ taken together with the nitrogen to which M is bonded and the carbon to which $R_6$ is bonded form a saturated unsubstituted heterocyclic group containing 3 or 4 ring carbon atoms;

Y is hydroxy, alkoxy, amino, or substituted amino, $Z_1$ and $Z_2$ each is a chemical bond, the group $X_3$—$(CH_2)_m$—, an amino acid group or the group $X_3$—$(CH_2)_m$—$X_3$ wherein $X_3$ is oxygen, sulfur or NH and m is an integer from 0 to 4 inclusive;

A and B each is the same as $R_1$ or halogen, trifluoromethyl, $OR_1$, $NO_2$, $NR_1R_2$, $SO_2NH_2$, or $SR_1$;

C is hydrogen alkyl, amino, —$CR_1$=$CR_2$—,

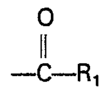

aminoalkyl, halogen, 2-furanylmethylamino, aminoaryl or aminobenzyl with the proviso that when $Z_2$ is sulfur and $Z_1$ is a chemical bond, then A, B and C cannot all be hydrogen; and salts, particularly pharmaceutically acceptable salts thereof.

2. A compound of Claim 1 wherein $R_1$, $R_2$, $R_3$ and $R_4$ each is hydrogen or a lower alkyl group; n is 1; M is cycloalkyl; one of $X_1$ and $X_2$ is —$CH_2$— and the other is —$CH_2$—$CHR_1$; Y is hydroxy or alkoxy; $Z_1$ is a chemical bond or the group —$OCH_2$—; $Z_2$ is sulfur; A and B each is hydrogen, halogen or the group —$SO_2NH_2$; and C is halogen, amino,

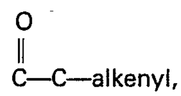

aminoalkyl, 2-furanylmethylamino, aminoaryl or aminobenzyl.

3. A compound of Claims 1 and 2 wherein at least one of $R_1$, $R_2$, $R_3$ and $R_4$ is methyl.

4. A compound of Claims 1—3 which is N-[3-(2,3-dichloro-4-(2-methylenė-1-oxobutyl)phenoxy] aceylthio-2-methylpropanoyl]-N-cyclopentyl-glycene methyl-ester.

5. A compound of Claims 1—3 which is N-[3-[2,3-dichloro-4-(2-methylene-1-oxobutyl)phenoxy]-acetylthio-2-methylpropanoyl]-N-cyclopentyl-glycine-t-butyl-ester.

6. A compound of Claims 1—3 which is N-[3-[2,3-dichloro-4-(2-methylene-1-oxobutyl)phenoxy]-acetylthio-2-methylpropanoyl]-N-cyclopentyl-glycine.

7. A compound of Claims 1—3 which is N-[3-(4-chloro-2-[(2-furanylmethyl)amino]-5-sulfamoyl)-benzoylthio-2-methylpropanoyl]-N-cyclopentyl-glycine-t-butyl-ester.

8. A compound of Claims 1—3 which is N-[3-(4-chloro-2-[(2-furanylmethyl)amino]-5-sulfamoyl)-benzoylthio-2-methylpropanoyl]-N-cyclopentyl-glycine.

9. A method for preparing a compound of formula (I) according to claims 1—8 which comprises reacting under acylating conditions an acylating derivative of an acid of formula (II)

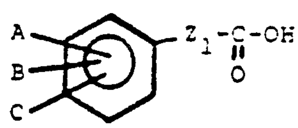

(II)

with a compound of formula (III)

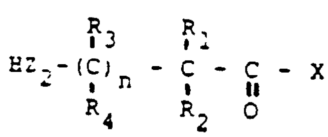

(III)

or an acrylating derivative of an acid of formula (IV)

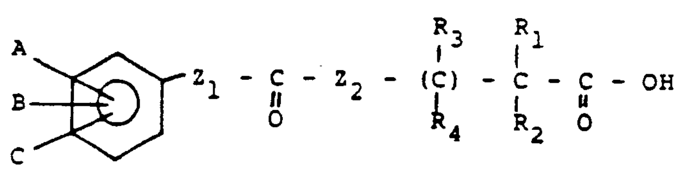

(IV)

with a compound of formula (V)

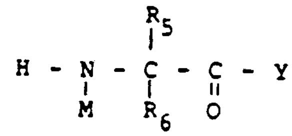

(V)

wherein X, $R_1$, $R_2$, $R_3$, $R_4$, $R_5$, $R_6$, n, M, $Z_1$, $Z_2$, A, B and C have the meanings given in claims 1—8 and optionally forming salts of the resulting products, especially pharmaceutically acceptable salts thereof.

10. A therapeutic composition comprising, in combination with at least one non-toxic pharmaceutically acceptable extended, an angiotensin converting enzyme inhibiting the diuresis-inducing amount of at least one compound of formula (I) in accordance with any of Claims 1—8.

**Claims for the Contracting State: AT**

1. A method for preparing a compound of the general formula

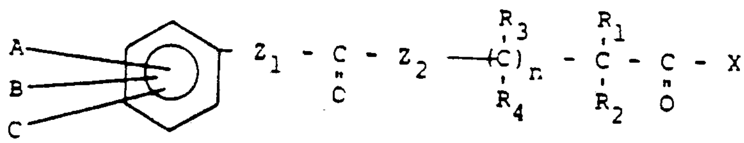

(I)

wherein X is

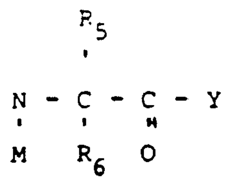

;

$R_1$, $R_2$, $R_3$, $R_4$, $R_5$, and $R_6$ are hydrogen, alkyl, alkenyl, alkynyl, phenyl-alkyl, and cycloalkyl, and may be the same or different;

n is an integer from 0 to 4 inclusive;

M is alkenyl, alkynyl, cycloalkyl, cycloalkyl-alkyl, polycycloalkyl, polycycloalkyl-alkyl, or M and $R_6$ taken together with the nitrogen to which M is bonded and the carbon to which $R_6$ is bonded form a saturated unsubstituted heterocyclic group containing 3 or 4 ring carbon atoms;

Y is hydroxy, alkoxy, amino, or substituted amino, $Z_1$ and $Z_2$ each is a chemical bond, the group $X_3(CH_2)_m$—, an amino acid group or the group $X_3$—$(CH_2)_m$—$X_3$ wherein $X_3$ is oxygen, sulfur or NH and m is an integer from 0 to 4 inclusive;

A and B each is the same as $R_1$ or halogen, trifluoromethyl, $OR_1$, $NO_2$, $NR_1R_2$, $SO_2NH_2$, or $SR_1$;

C is hydrogen alkyl, amino, —$CR_1$=$CR_2$—,

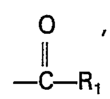

,

aminoalkyl, halogen, 2-furanylmethylamino, aminoaryl or aminobenzyl with the proviso that when $Z_2$ is sulfur and $Z_1$ is a chemical bond, then A, B and C cannot all be hydrogen; which comprises reacting under acylating conditions an acylating derivative of an acid of formula (II)

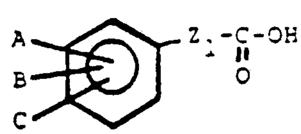

(II)

with a compound of formula (III)

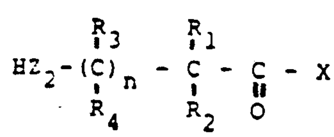

(III)

or an acylating derivative of an acid of formula (IV)

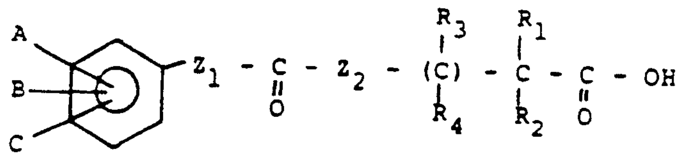

(IV)

with a compound of formula (V)

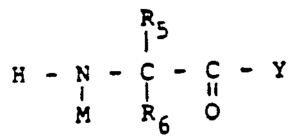

(V)

wherein X, $R_1$, $R_2$, $R_3$, $R_4$, $R_5$, $R_6$, n, M, $Z_1$, $Z_2$, A, B and C have the above meanings, and optionally forming salts of the resulting products, especially pharmaceutically acceptable salts thereof.

2. The method according to Claim 1 wherein $R_1$, $R_2$, $R_3$ and $R_4$ ea07, $R_2$, $R_3$ and $R_4$ each is hydrogen or a lower alkyl group; n is 1; M is cycloalkyl; one of $X_1$ and $X_2$ is $—CH_2—$ and the other is $—CH_2—CHR_1—$; Y is hydroxy or alkoxy; $Z_1$ is a chemical bond or the group $—OCH_2—$; $Z_2$ is sulfur; A and B each is hydrogen, halogen or the group $—SO_2NH_2$; and C is halogen, amino,

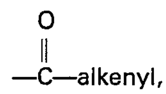

aminoalkyl, 2-furanylmethylamino, aminoaryl or aminobenzyl.

3. The method according to Claims 1 and 2 wherein at least one of $R_1$, $R_2$, $R_3$ and $R_4$ is methyl.

4. The method according to Claims 1—3 wherein the compound is N-[3-[2,3-dichloro-4-(2-methylene-1-oxobutyl)phenoxy] acetylthio-2-methylpropanoyl]-N-cyclopentyl-glycine-methyl-ester.

5. The method according to Claims 1—3 wherein the compound is N-[3-[2,3-dichloro-4-(2-methylene-1-oxobutyl)phenoxy]-N-acetylthio-2-methylpropanoyl]-N-cyclopentyl-glycine-t-butyl-ester.

6. The method according to Claims 1—3 wherein the compound is N-[3-[2,3-dichloro-4-(2-methylene-1-oxobutyl)phenoxy]-acetylthio-2-methylpropanoyl]-N-cyclopentyl-glycine.

7. The method according to Claims 1—3 wherein the compound is N-[3-(4-chloro-2-[(2-furanylmethyl)-amino]-5-sulfamoyl)-benzoylthio-2-methylpropanoyl]-N-cyclopentyl-glycine-t-butyl-ester.

8. The method according to Claims 1—3 wherein the compound is N-[3-(4-chloro-2-[(2-furanylmethyl)-amino]-5-sulfamoyl)-benzoylthio-2-methylpropanoyl]-N-cyclopentyl-glycine.

9. A method for preparing a therapeutic composition by combining at least one non-toxic pharmaceutically acceptable extender with an angiotensin converting enzyme inhibiting and diuresis-inducing amount of at least one compound of formula (I) prepared in accordance with any of Claims 1—8.

**Patentansprüche für die Vertragsstaaten: BE CH DE FR GB IT LI LU NL SE**

1. Verbindungen der allgemeinen Formel

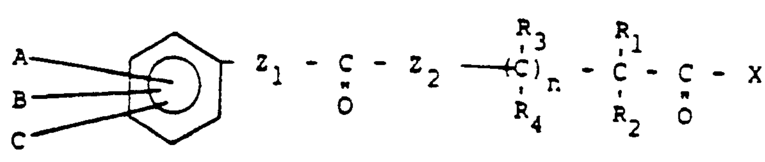

(I)

worin bedeuten:

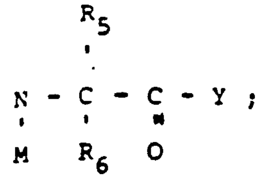

X

$R_1$, $R_2$, $R_3$, $R_4$, $R_5$ und $R_6$, die gleich oder verschieden sein können, Wasserstoff, Alkyl, Alkenyl, Alkinyl, Phenyl-alkyl und Cycloalkyl;

n ein ganze Zahl von 0 bis einschließlich 4;

M Alkenyl, Alkinyl, Cycloalkyl, Cycloalkyl-alkyl, Polycycloalkyl, Polycycloalkyl-alkyl oder M und $R_6$ bilden zusammen mit dem Stickstoff, an den M gebunden ist und dem Kohlenstoff, an den $R_6$ gebunden ist, eine gesättigte, unsubstituierte heterocyclische Gruppe mit 3 oder 4 Ringkohlenstoffatomen;

Y Hydroxy, Alkoxy, Amino oder substituiertes Amino;

$Z_1$ und $Z_2$ jeweils eine chemische Bindung, die Gruppe $X_3—(CH_2)_m—$, eine Aminosäuregruppe oder die Gruppe $X_3—(CH_2)_m—_3$, worin $X_3$ Sauerstoff, Schwefel oder NH und m eine ganze Zahl von 0 bis einschließlich 4 bedeuten;

A und B jeweils das gleiche wie $R_1$ oder Halogen, Trifluoromethyl, $OR_1$, $NO_2$, $NR_1R_2$, $SO_2NH_2$ oder $SR_1$;

C Wasserstoff, Alkyl, Amino, $—CR_1{=}CR_2—$,

$$—\overset{\overset{O}{\|}}{C}—R_1$$

Aminoalkyl, Halogen, 2-Furanylmethylamino, Aminoaryl oder Aminobenzyl, mit der Maßgabe, daß, wenn $Z_2$ Schwefel und $Z_1$ eine chemische Bindung bedeuten, A, B und C nicht alle Wasserstoff sein können; und Salze, insbesondere pharmazeutisch annehmbare Salze hiervon.

2. Verbindung nach Anspruch 1, wobei $R_1$, $R_2$, $R_3$ und $R_4$ jeweils Wasserstoff oder eine niedere Alkylgruppe; n = 1; M Cycloalkyl; eines von $X_1$ und $X_2$ $—CH_2—$ und das andere $—CH_2—CHR_1—$; Y Hydroxy oder Alkoxy; $Z_1$ eine chemische Bindung oder die Gruppe $—OCH_2—$; $Z_2$ Schwefel; A und B jeweils Wasserstoff, Halogen oder die Gruppe $—SO_2NH_2$; und C Halogen, Amino,

$$—\overset{\overset{O}{\|}}{C}\text{-Alkenyl},$$

Aminoalkyl, 2-Furanylmethylamino, Aminoaryl oder Aminobenzyl bedeuten.

3. Verbindung nach den Ansprüchen 1 und 2, wobei mindestens eines von $R_1$, $R_2$, $R_3$ und $R_4$ Methyl bedeutet.

4. Verbindung nach den Ansprüchen 1 bis 3, wobei es sich um den N-[3-[2,3-Dichloro-4-(2-methylen-1-oxobutyl)phenoxy]acetylthio-2-methylpropanoyl]-N-cyclopentyl-glycin-methylester handelt.

5. Verbindung nach den Ansprüchen 1 bis 3, wobei es sich um den N-[3-[2,3-Dichlor-4-(2-methylen-1-oxobutyl)phenoxy]acetylthio-2-methylpropanoyl]-N-cyclopentyl-glycin-t-butylester handelt.

6. Verbindung nach den Ansprüchen 1 bis 3, wobei es sich um das N-[3-[2,3-Dichlor-4-(2-methylen-1-oxobutyl)phenoxy]acetylthio-2-methylpropanoyl]-N-cyclopentyl-glycin handelt.

7. Verbindung nach den Ansprüchen 1 bis 3, wobei es sich um den N-[3-(4-Chlor-2-[(2-Furanylmethyl)-amino]-5-sulfamoyl)benzoylthio-2-methylpropanoyl]-N-cyclopentyl-glycin-t-butylester handelt.

8. Verbindung nach den Ansprüchen 1 bis 3, wobei es sich um das N-[3-(4-Chlor-2-[(2-Furanylmethyl)-amino]-5-sulfamoyl)benzoylthio-2-methylpropanoyl]-N-cyclopentyl-glycin handelt.

9. Verfahren zur Herstellung einer Verbindung der Formel (I) nach den Ansprüchen 1 bis 8, umfassend das Umsetzen unter Acylierungsbedingungen eines Acyliergungs-Derivates einer Säure der Formel (II)

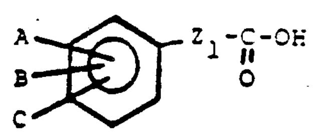

(II)

mit einer Verbindung der Formel (III)

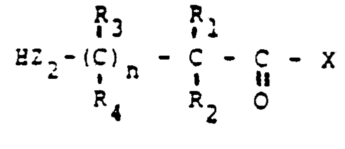

(III)

oder eines Acylierungs-Derivates einer Säure der Formel (IV)

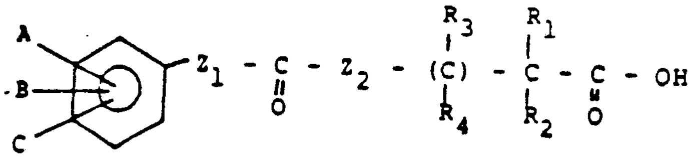
(IV)

mit einer Verbindung der formel (V)

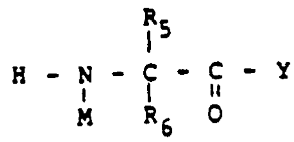
(V)

worin X, $R_1$, $R_2$, $R_3$, $R_4$, $R_5$, $R_6$, n, M, $Z_1$, $Z_2$, A, B und C die in den Ansprüchen 1 bis 8 angegebenen Bedeutungen besitzen und wahlweise Bilden von Salzen der resultierenden Produkte, insbesondere pharmazeutisch annehmbaren Salzen hiervon.

10. Arzneimittel, umfassend, in Kombination mit mindestens einem nichttoxischen, pharmazeutisch annehmbaren Streckmittel, eine das Angiotensin umwandelnde Enzym inhibierende und die Diurese induzierende Menge mindestens einer Verbindung der Formel (I) gemäß einem der Ansprüche 1 bis 8.

**Patentansprüche für den Vertragsstaat: AT**

1. Verfahren zur Herstellung einer Verbindung der allgemeinen Formel

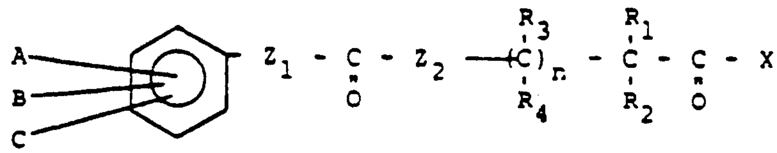
(I)

worin bedeuten:

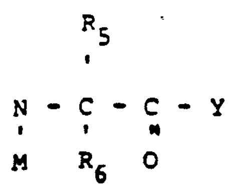
;

X

$R_1$, $R_2$, $R_3$, $R_4$, $R_5$ und $R_6$, die gleich oder verschieden sein können, Wasserstoff, Alkyl, Alkenyl, Alkinyl, Phenyl-alkyl und Cycloalkyl;

n ein ganze Zahl von 0 bis einschließlich 4;

M Alkenyl, Alkinyl, Cycloalkyl, Cycloalkyl-alkyl, Polycycloalkyl, Polycycloalkyl-alkyl oder M und $R_6$ bilden zusammen mit dem Stickstoff, an den M gebunden ist und dem Kohlenstoff, an den $R_6$ gebunden ist, eine gesättigte, unsubstituierte heterocyclische Gruppe mit 3 oder 4 Ringkohlenstoffatomen;

Y Hydroxy, Alkoxy, Amino oder substituiertes Amino;

$Z_1$ und $Z_2$ jeweils eine chemische Bindung, die Gruppe $X_3—(CH_2)_m—$, eine Aminosäuregruppe oder die Gruppe $X_3—(CH_2)_m—_3$, worin $X_3$ Sauerstoff, Schwefel oder NH und m eine ganze Zahl von 0 bis einschließlich 4 bedeuten;

A und B jeweils das gleiche wie $R_1$ oder Halogen, Trifluoromethyl, $OR_1$, $NO_2$, $NR_1R_2$, $SO_2NH_2$ oder $SR_1$;

C Wasserstoff, Alkyl, Amino, $—CR_1{=}CR_2—$,

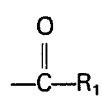

Aminoalkyl, Halogen, 2-Furanylmethylamino, Aminoaryl oder Aminobenzyl, mit der Maßgabe, daß, wenn $Z_2$ Schwefel und $Z_1$ eine chemische Bindung bedeuten, A, B und C nicht alle Wasserstoff sein können;

umfassend das Umsetzen unter Acylierungsbedingungen eines Acylierungs-Derivates einer Säure der Formel (II)

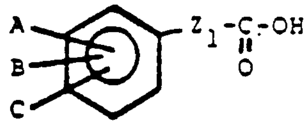

(II)

mit einer Verbindung der Formel (III)

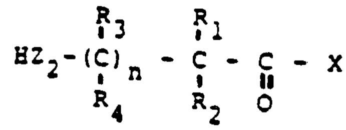

(III)

oder eines Acylierungs-Derivates einer Säure der Formel (IV)

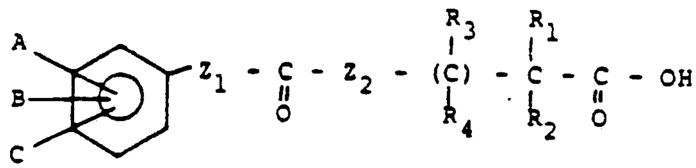

(IV)

mit einer Verbindung der Formel (V)

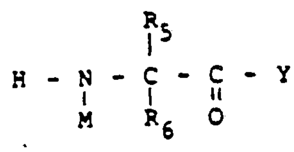

(V)

worin X, $R_1$, $R_2$, $R_3$, $R_4$, $R_5$, $R_6$, n, M, $Z_1$, $Z_2$, A, B und C die oben angegebenen Bedeutungen besitzen, und wahlweise Bilden von Salzen der resultierenden Produkte, insbesondere pharmazeutisch annehmbaren Salzen hiervon.

2. Verbindung nach Anspruch 1, wobei $R_1$, $R_2$, $R_3$ und $R_4$ jeweils Wasserstoff oder eine niedere Alkylgruppe; n = 1; M Cycloalkyl; eines von $X_1$ und $X_2$ $-CH_2-$ und das andere $-CH_2-CHR_1-$; Y Hydroxy oder Alkoxy; $Z_1$ eine chemische Bindung oder die Gruppe $-OCH_2-$; $Z_2$ Schwefel; A und B jeweils Wasserstoff, Halogen oder die Gruppe $-SO_2NH_2$; und C Halogen, Amino,

$$-\overset{\overset{\displaystyle O}{\|}}{C}\text{-Alkenyl},$$

Aminoalkyl, 2-Furanylmethylamino, Aminoaryl oder Aminobenzyl bedeuten.

3. Verbindung nach den Ansprüchen 1 und 2, wobei mindestens eines von $R_1$, $R_2$, $R_3$ und $R_4$ Methyl bedeutet.

4. Verfahren nach den Ansprüchen 1 bis 3, wobei es sich bei der Verbindung um den N - [3 - [2,3 - Dichloro - 4 - (2 - methylen - 1 - oxobutyl)phenoxy]acetylthio - 2 - methylpropanoyl] - N - cyclopentyl - glycin - methylester handelt.

5. Verfahren nach den Ansprüchen 1 bis 3, wobei es sich bei der Verbindung umd den N - [3 - [2,3 - Dichlor - 4 - (2 - methylen - 1 - oxobutyl)phenoxy]acetylthio - 2 - methylpropanoyl] - N - cyclopentyl - glycin - t - butylester handelt.

6. Verfahren nach den Ansprüchen 1 bis 3, wobei es sich bei der Verbindung um das N-[3-[2,3-Dichlor-4-(2-methylen-1-oxobutyl)phenoxy]acetylthio-2-methylpropanoyl]-N-cyclopentyl-glycin handelt.

7. Verfahren nach den Ansprüchen 1 bis 3, wobei es sich bei der Verbindung um den N - [3 - (4 - Chlor - 2 - [(2 - Furanylmethyl)amino] - 5 - sulfamoyl)benzoylthio - 2 - methylpropanoyl] - N - cyclopentyl - glycin - t - butylester handelt.

8. Verfahren nach den Ansprüchen 1 bis 3, wobei es sich bei der Verbindung um den N-[3-(4-Chlor-2-[(2-Furanylmethyl)amino]-5-sulfamoyl)benzoylthio-2-methylpropanoyl]-N-cyclopentyl-glycin handelt.

9. Verfahren zur Herstellung eines Arzneimittels durch Kombinieren mindestens eines nichttoxischen, pharmazeutisch annehmbaren Streckmittels mit einer das Angiotensin umwandelnde Enzyminhibierenden und die Diurese induzierenden Menge mindestens einer nach einem der Ansprüche 1 bis 8 hergestellten Verbindung der Formel (I).

**Revendications pour les Etats contractants: BE CH DE FR GB IT LI LU NL SE**

1. Composé de la formule générale:

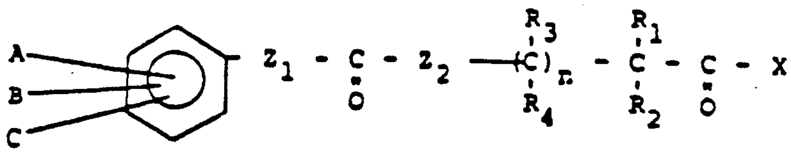

(I)

dans laquelle

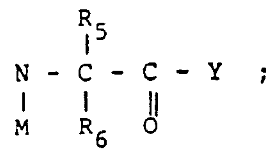

X est

$R_1$, $R_2$, $R_3$, $R_4$. $R_5$ et $R_6$ sont des atomes d'hydrogène, des groupes alkyle, alcényle, alcynyle, phénylalkyle et cycloalkyle et peuvent être semblables ou différents;

n est un nombre entier de 0 à 4 inclusivement;

M est un groupe alcényle, alcynyle, cycloalkyle, cycloalkylalkyle, polycyclo-alkyle, polycycloalkylalkyle, ou M et $R_6$ pris ensemble avec l'azote auquel M est rattaché et le carbone auquel $R_6$ est rattaché forment un groupe hétérocyclique non substitué saturé contenant 3 ou 4 atomes de carbone dans le noyau;

Y est un groupe hydroxyle, alcoxyle, amine ou amine substitué, $Z_1$ et $Z_2$ sont chacun une liaison chimique, le groupe $X_3$—$(CH_2)_m$—, un groupe aminoacide ou le groupe $X_3$—$(CH_2)$—$X_3$ dans lequel $X_3$ est l'oxygène, le soufre ou NH et m est un nombre entier de 0 à 4 inclusivement;

A et B sont chacun la même chose que $R_1$ ou un halogène, un groupe trifluorométhyl, $OR_1$, $NO_2$, $NR_1R_2$, $SO_2NH_2$ ou $SR_1$;

C est l'hydrogène, un groupe alkyle, amine, —$CR_1$=$CR_2$—,

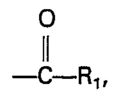

aminoalkyle, un halogène, un groupe 2-furannylméthylamine, aminoacryle ou aminobenzyle, étant entendu que, quand $Z_2$ est le soufre et que $Z_1$ est une liaison chimique, A, B et C ne peuvent pas être tous l'hydrogène;

et des sels, particulièrement des sels pharmaceutiquement acceptables de ceux-ci.

2. Composé selon la revendication 1, dans lequel $R_1$, $R_2$, $R_3$ et RI $R_1$, $R_2$, $R_3$ et $R_4$ sont chacun l'hydrogène ou un groupe alkyle inférieur; n est 1; M est un groupe cycloalkyle, l'un de $X_1$ et $X_2$ est —$CH_2$— et l'autre est —$CH_2CHR_1$—; Y est un groupe hydroxyle ou alcoxyle; $Z_1$ est une liaison chimique ou le groupe —$OCH_2$; $Z_2$ est le soufre; A et B sont chacun l'hydrogène, un halogène ou le groupe —$SO_2NH_2$; et C est un halogène, un groupe amine,

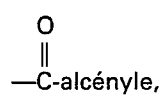

aminoalkyle, 2-furannylméthylamine, aminoaryle ou aminobenzyle.

3. Composé selon les revendications 1 et 2, dans lequel au moins un de $R_1$, $R_2$, $R_3$ et $R_4$ est un groupe méthyle.

4. Composé selon les revendications 1 à 3, qui est l'ester méthylique de la N-[3-[2,3-dichloro-4-(2-méthylène-1-oxobutyl)-phénoxy]-acétylthio-2-méthylpropanoyl]-N-cyclopentylglycine.

5. Composé selon les revendications 1 à 3, qui est l'ester butylique tertiaire de la N-[3-[2,3-dichloro-4-(2-méthylène-1-oxobutyl)-phénoxy]-acétylthio-2-méthylpropanoyl]-N-cyclopentylglycine.

6. Composé selon les revendications 1 à 3, qui est la N-[3-[2,3-dichloro-4-(2-méthylène-1-oxobutyl)-phénoxy]-acétylthio-2-méthylpropanoyl]-N-cyclopentylglycine.

7. Composé selon les revendications 1 à 3, qui est l'ester butylique tertiaire de la N-[3-(4-chloro-2-[(2-furannylméthyl)-amino]-5-sulfamoyl)-benzoylthio-2-méthylpropanoyl]-N-cyclopentylglycine.

8. Composé selon les revendications 1 à 3, qui est la N-[3-(4-chloro-2-[(2-furannylméthyl)amino]-5-sulfamoyl)-benzoylthio-2-méthylpropanoyl]-N-cyclopentylglycine.

9. Procédé de préparation d'un composé de la formule (I) selon les revendications 1 à 8, qui consiste à faire réagir, dans les conditions d'acylation, un dérivé acylant d'un acide de la formule (II)

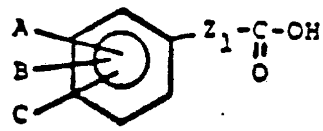

(II)

sur un composé de la formule (III)

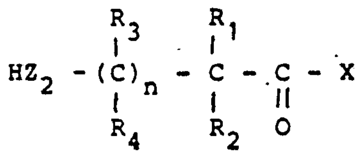

(III)

ou un dérivé acylant d'un acide de la formule (IV)

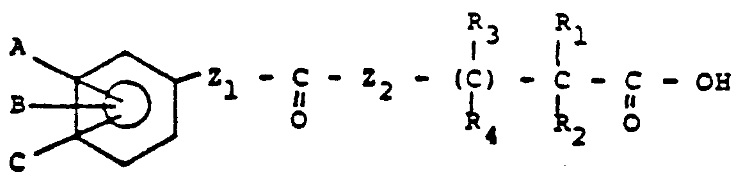

(IV)

sur un composé de la formule (V)

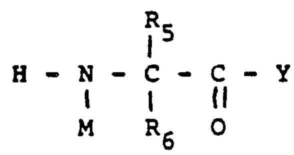

(V)

dans lesquelles X, $R_1$, $R_2$, $R_3$, $R_4$, $R_5$, $R_6$, n, M, $Z_1$, $Z_2$, A, B et C ont les significations données aux revendications 1 à 8 et, facultativement, à former des sels des produits obtenus, spécialement des sels pharmaceutiquement acceptables de ceux-ci.

10. Composition thérapeutique comprenant, en association avec un moins un extendeur non toxique pharmaceutiquement acceptable, au moins un composé de la formule (I) selon l'une quelconque des revendications 1 à 8, en quantité inhibant l'enzyme convertisseuse de l'angiotensine et provoquant la diurèse.

**Revendications pour l'Etat contractant: AT**

1. Procédé de préparation d'un composé de la formule générale

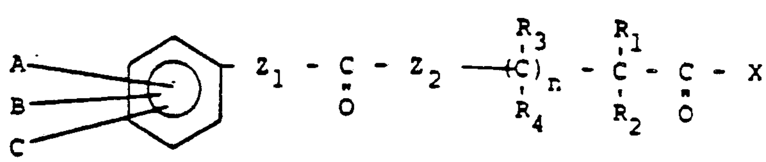

(I)

dans laquelle

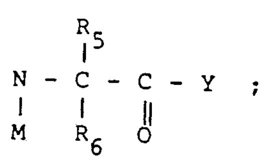

X est

$R_1$, $R_2$, $R_3$, $R_4$, $R_5$ et $R_6$ sont des atomes d'hydrogène, des groupes alkyle, alcényle, alcynyle, phénylalkyle et cycloalkyle et peuvent être semblables ou différents;

n est un nombre entier de 0 à 4 inclusivement;

M est un groupe alcényle, alcynyle, cycloalkyle, cycloalkyl-alkyle, polycycloalkyle, polycycloalkyl-alkyle, ou M est $R_6$ pris ensemble avec l'azote auquel M est rattaché et le carbone auquel $R_6$ est rattaché forment un groupe hétérocyclique non substitué saturé contenant 3 ou 4 atomes de carbone dans le noyau;

Y est un groupe hydroxyle, alcoxyle, amine ou amine substitué, $Z_1$ et $Z_2$ sont chacun une liaison chimique, le groupe $X_3-(CH_2)_m-$, un groupe aminoacide ou le groupe $X_3(CH_2)_m-X_3$ dans lequel $X_3$ est l'oxygène, le soufre ou NH et m est un nombre entier de 0 à 4 inclusivement;

A et B sont chacun la même chose que $R_1$ ou un halogène, un groupe trifluorométhyle, $OR_1$, $NO_2$, $NR_1R_2$, $SO_2NH_2$ ou $SR_1$;

C est l'hydrogène, un groupe alkyle, amine, $—CR_1=CR_2—$,

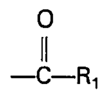

aminoalkyle, un halogène, un groupe 2-furannylméthylamine, aminoaryle ou aminobenzyle, étant entendu que, quand $Z_2$ est le soufre et que $Z_1$ est une liaison chimique, A, B et C ne peuvent pas être tous l'hydrogène;

qui consiste à faire réagir, dans des conditions d'acylation, un dérivé acylant d'un acide de la formule (II)

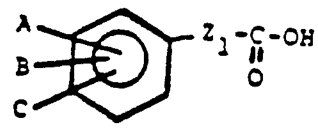

(II)

sur un composé de la formule (III)

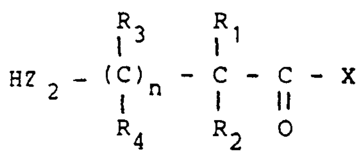

(III)

ou un dérivé acylant d'un acide de la formule (IV)

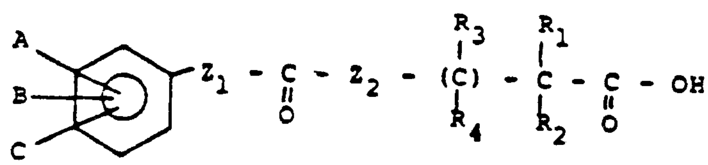

(IV)

sur un composé de la formule (V)

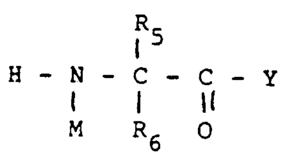

(V)

dans lesquelles X, $R_1$, $R_2$, $R_3$, $R_4$, $R_5$, $R_6$, n, M, $Z_1$, $Z_2$, A, B et C ont les significations données ci-dessuset, facultativement, à former des sels des produits obtenus, spécialement des sels pharmaceutiquement acceptables de ceux-ci.

2. Procédé selon la revendication 1, dans lequel $R_1$, $R_2$, $R_3$, et $R_4$ sont chacun l'hydrogène ou un groupe alkyle inférieur; n est 1; M est un groupe cycloalkyle; l'un de $X_1$ et $X_2$ est $—CH_2—$ et l'autre est $—CH_2—CHR_1—$; Y est un groupe hydroxyle ou alcoxyle; $Z_1$ est une liaison chimique ou le groupe $—OCH_2$; $Z_2$ est le soufre; A et B sont chacun l'hydrogène, un halogène ou le groupe $—SO_2NH_2$; et C est un halogène, un groupe amine,

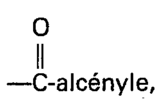

aminoalkyle, 2-furannylméthylamine, aminoaryle ou aminobenzyle.

3. Procédé selon les revendications 1 et 2, dans lequel au moins un de $R_1$, $R_2$, $R_3$ et $R_4$ est une groupe méthyle.

4. Procédé selon les revendications 1 à 3, dans lequel le composé est l'ester méthylique de la N-[3-[2,3-dichloro-4-(2-méthylène-1-oxobutyl)-phénoxy]-acétylthio-2-méthylpropanoyl]-N-cyclopentylglycine.

5. Procédé selon les revendications 1 à 3, dans lequel le composé est l'ester butylique tertiaire de la N-[3 - [2,3 - dichloro - 4 - (2 - méthylène - 1 - oxobutyul) - phénoxy]-acétylthio - 2 - méthylpropanoyl] - N - cyclopentylglycine.

6. Procédé selon les revendications 1 à 3, dans lequel le composé est la N-[3-[2,3-dichloro-4-(2-méthylène-1-oxobutyl)-phénoxy]-acétylthio-2-méthylpropanoyl]-N-cyclopentylglycine.

7. Procédé selon les revendications 1 à 3, dans lequel le composé est l'ester butylique tertiaire de la N-[3 - (4 - chloro - 2 - [(2 - furannylméthyl) - amino] - 5 - sulfamoyl) - benzoylthio - 2 - méthylpropanoyl] - N - cyclopentylglycine.

8. Procédé selon les revendications 1 à 3, dans lequel le composé est la N-[3-(4-chloro-2-[(2-furannylméthyl)amino]-5-sulfamoyl)-benzoylthio-2-méthylpropanoyl]-N-cyclopentylglycine.

9. Procédé de préparation d'une composition thérapeutique, en associant au moins un extendeur non toxique pharmaceutiquement acceptable à au moins un composé de la formule (I), préparé selon l'une quelconque des revendications 1 à 8, en quantité inhibant l'enzyme convertisseuse de l'angiotensine et provoquant la diurèse.